# EUROPÄISCHE PATENTSCHRIFT

(11) **EP 0 333 063 B1**
(45) Veröffentlichungstag und Bekanntmachung des Hinweises auf die Patenterteilung: **24.11.1993**
(21) Anmeldenummer: 89104280.6
(22) Anmeldetag: 10.03.1989
(51) Int. Cl.: G01N 5/04, G01N 33/42

(54) **Verfahren und Vorrichtung zur Ermittlung des Bindemittelgehalts von bituminösen Baustoffen**
Method and apparatus for determining the binding materials content of bituminous road-making materials
Procédé et dispositif de détermination de la teneur en liants de matériaux de construction bitumineux

(30) Priorität: 17.03.1988 DE 3808888
(43) Veröffentlichungstag der Anmeldung: 20.09.1989
(73) Patentinhaber: HERMANN RIEDE STRASSEN- UND TIEFBAU GMBH & CO. KG, D-34059 Kassel (DE)
(72) Erfinder: Schneider, Ulrich, Prof. Dr.-Ing. habil., D-3503 Lohfelden (DE)
(74) Vertreter: Freiherr von Schorlemer, Reinfried, Dipl.-Phys.

(56) Entgegenhaltungen:
- DD-A- 113 407
- FR-A- 1 492 198
- US-A- 1 967 424
- US-A- 2 679 159
- US-A- 3 453 083
- H.F. MARK et al.:Encyclopedia of Polymer Science and Technology, Bd. 14, Seite 1 ff

## Beschreibung

Die Erfindung betrifft ein Verfahren der im Oberbegriff des Anspruchs 1 angegebenen Gattung.

Die Entwicklung derartiger Verfahren ist eine Folge der in letzter Zeit immer deutlicher werdenden Verknappung an Rohrstoffen und des häufig hohen Anfalls an nicht nutzbarem Altmaterial. Im Straßenbau wird bereits vielfach gefordert, bei der Ausbesserung alter oder bei der Herstellung neuer Straßenbeläge das anfallende Altmaterial wiederzuverwenden, indem es beispielsweise den üblichen frischen Gemischen aus Gesteinen (z.B. Basalt, Sand od. dgl.), Zuschlagstoffen bzw. Füllern (z.B. Kalkstein) und Bindemitteln (in der Regel reines Bitumen) zusätzlich beigegeben und zusammen mit diesen in einer speziellen Trocken- und Mischanlage zu Asphaltheißmischgut verarbeitet wird (DE-OS 36 16 995).

Ein durch Zugabe von Altmaterial hergestellter, bituminöser Baustoff muß den gleichen Qualitätsanforderungen wie ein ohne die Zugabe von Altmaterial hergestellter Baustoff genügen. Um diesen Anforderungen entsprechen zu können, ist es erforderlich, die Zusammensetzung des Altmaterials hinsichtlich des Bindemittelgehalts, der Kornverteilung und des Gehalts an Zuschlagstoffen zu kennen, da nur dann die mengenmäßige Zugabe der frischen Ausgangsstoffe einerseits und des Altmaterials andererseits exakt gesteuert werden kann.

Die Ermittlung des Bindemittelgehalts ist durch Anwendung des eingangs bezeichneten, bekannten Verfahrens möglich (DD - A - 113 407), bei dem zwei Teilproben eines bituminösen Bindemittelgesteinsgemisches auf 600°C erhitzt und nach Abkühlung auf Zimmertemperatur auf 0,01 g genau gewogen werden. Von dem dadurch erhaltenen Mittelwert wird der Mittelwert einer Nullprüfung abgezogen, die bei Mischbeginn und bei Rezepturveränderungen in gleicher Weise mit zwei Teilproben des Mineralgemisches durchgeführt wird. Voraussetzung für eine einigermaßen genaue Messung ist daher, daß die beiden Proben jeweils entweder vollkommen trocken sind oder denselben Feuchtigkeitsanteil aufweisen.

Zur Bestimmung des Bindemittelgehalts von Altasphalten ist das bekannte Verfahren ungeeignet, da es die Kenntnis ihrer Mineralgehalte bei der Nullprüfung voraussetzt, diese aber grundsätzlich als unbekannt anzunehmen sind. Außerdem können Altbaustoffe je nach Klima- und/oder Wetterlage unterschiedliche Anteile an Feuchtigkeit enthalten, so daß es möglich sein muß, den auf den Feuchtigkeitsverlust entfallenden Anteil des Gesamtgewichtsverlusts ausreichend genau von dem auf den Bindemittelabbau entfallenden Anteil zu trennen. Auch dies ist bei Anwendung des bekannten Verfahrens nicht möglich.

Der Erfindung liegt daher die Aufgabe zugrunde, das eingangs bezeichnete Verfahren dahingehend zu verbessern, daß es auch bei der Ermittlung des Bindemittelgehalts von Altasphalten angewendet werden kann und eine Abtrennung des auf die Feuchtigkeit entfallenden Gewichtsanteils ermöglicht.

Zur Lösung dieser Aufgabe dienen die kennzeichnenden Merkmale des Anspruchs 1.

Die nachfolgend allgemein als ''thermischer Abbau'' bezeichnete Entfernung der bituminösen Komponente kann einerseits bei Anwesenheit von Sauerstoff als Verbrennung, andererseits bei Abwesenheit von Sauerstoff als thermische Zersetzung bezeichnet werden. Dabei dürften in praktischen Anwendungsfällen beide Arten des Abbaus nebeneinander auftreten. Weiterhin beruht die Erfindung auf der Erkenntnis, daß sich aus einer Kurve, die den Gewichtsverlust der Probe während des thermischen Abbaus in Abhängigkeit von der Zeit wiedergibt, sowohl die auf die Feuchtigkeit als auch die auf das Bindemittel entfallenden Gewichtsanteile ermitteln lassen. Schließlich ergibt sich der Vorteil, daß die übrigen Bestandteile der hier interessierenden Baustoffe bei Einhaltung charakteristischer Temperaturbereiche weder zersetzt oder verbrannt noch in anderer Weise schädlich beeinflußt werden, so daß die Analyse der übrigen Bestandteile auf üblichem Wege nach der Entfernung der Bindemittelkomponente keinerlei Schwierigkeiten bereitet.

Die Erfindung wird nachfolgend in Verbindung mit der beiliegenden Zeichnung an Ausführungsbeispielen näher erläutert. Es zeigen:
- Fig. 1: schematisch anhand eines Diagramms den Verlauf des Gewichtsverlusts einer Probe aus bituminösem Baustoff durch thermischen Abbau des Bindemittels nach ausreichender Vortrocknung der Probe;
- Fig. 2: eine Vorrichtung zur weitgehend automatischen Durchführung des erfindungsgemäßen Verfahrens; und
- Fig. 3: schematisch anhand eines weiteren Diagramms den Verlauf des Gewichtsverlusts einer Probe aus bituminösem Baustoff durch technischen Abbau des Bindemittels bei weitgehend automatischer Ausgestaltung des erfindungsgemäßen Verfahrens.

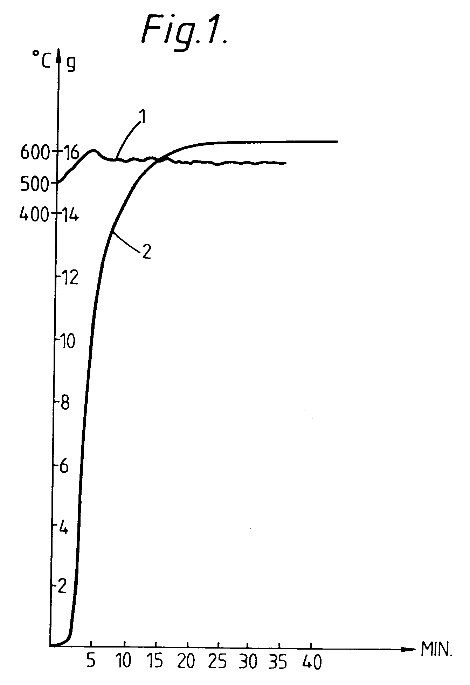

Fig. 1 zeigt schematisch den Verlauf des thermischen Abbaus des Bindemittels am Beispiel einer Probe aus 299,8 g Fräs-Asphalt. Die Probe wurde zunächst in üblicher Weise wenigstens einen Tag lang bei ca. 105 °C vorgetrocknet, um den Wasseranteil zu entfernen. Sodann wurde die Probe in einem elektrisch beheizten Ofen einer mittleren Temperatur von ca. 550 °C ausgesetzt. Dabei ist in Fig. 1 längs der Abszisse die Dauer der thermischen Behandlung in Minuten abgetragen, während längs der Ordinate im rechten Teil der gemessene Gewichtsverlust der Probe in Gramm und im linken Teil die im Ofen herrschende Temperatur abgetragen sind. Der durch eine Kurve 1 dargestellte, wellenförmige Temperaturverlauf ist dabei eine Folge der üblichen Temperaturregelung, die zu Temperaturschwankungen zwischen vorgewählten Mindest- und Höchstwerten führt.

Der durch eine Kurve 2 dargestellte Verlauf des Gewichtsverlusts der Probe zeigt, daß innerhalb der ersten ca. drei bis vier Minuten nur ein sehr geringer Gewichtsverlust eintritt. Danach nimmt der durch die einsetzende Zersetzung bzw. Verbrennung des bituminösen Bindemittels verwirkte Gewichtsverlust der Probe bis zu einer Versuchsdauer von ca. zwanzig Minuten steil zu, während anschließend praktisch kein Ansteigen des Gewichtsverlusts mehr zu beobachten ist. Daraus läßt sich schließen, daß nach ca. zwanzig bis fünfundzwanzig Minuten das gesamte in der Probe enthaltene Bindemittel zersetzt bzw. verbrannt und aus der Probe entfernt ist, so daß der thermische Abbau nach fünfundzwanzig Minuten als abgeschlossen betrachtet werden kann. Der Gewichtsverlust der Probe beträgt beim dargestellten Beispiel im übrigen 16,3 g, was einem Bindemittelgehalt der Ausgangsprobe von ca. 5,44 Gew.% entspricht.

Neben der schnelleren Entfernung des Bindemittels aus der Probe bringt das erfindungsgemäße Verfahren den weiteren Vorteil mit sich, daß der nach dem thermischen Abbau des Bindemittels verbleibende Rest der Probe aufgrund der vorhergehenden Erhitzung völlig trocken ist und daher sofort einer Siebanalyse unterworfen werden kann, woraus eine weitere Reduzierung der für die Gesamtanalyse benötigten Zeit resultiert. Es ist dabei allerdings vorteilhaft, das verbleibende Mineralgemisch nicht in heißem Zustand, sondern erst nach einer gewissen, vorzugsweise bis auf die Umgebungstemperatur vorgenommenen Abkühlung der Siebanalyse zu unterwerfen, um zu vermeiden, daß die Körner miteinander verkleben oder zu stark aneinander- bzw. an den Sieben haften und dadurch den Siebvorgang und insbesondere die Siebgeschwindigkeit beeinflussen. Beeinträchtigungen der Siebanalyse durch den vorhergehenden thermischen Abbau des Bitumens bzw. bituminösen Bindemittels konnten bisher nicht beobachtet werden. Im Bedarfsfall kann zusätzlich zur Siebanalyse noch eine Bestimmung des Gehalts an Zuschlagstoffen vorgesehen werden, sofern sich diese Bestimmung nicht ebenfalls durch Siebung erledigen läßt.

Bei der Analyse von Proben mit genau bekanntem Bindemittelgehalt hat sich weiter ergeben, daß die bisher bei der Vortrocknung üblicherweise verwendeten Temperaturen von ca. 105 °C zu gering bemessen sind, um den gesamten in der Probe enthaltenen Wasseranteil zu verdampfen, auch wenn die Vortrocknung über mehrere Tage durchgeführt wird. Daher wird erfindungsgemäß vorgeschlagen, die Vortrocknung bei größeren Temperaturen, vorzugsweise bei 200 bis 300 °C durchzuführen. Nach deren Anwendung über beispielsweise 24 Stunden konnten keine merklichen, auf die Freisetzung von Restfeuchtigkeit zurückführbaren Gewichtsverluste während des thermischen Abbaus des Bindemittels beobachtet werden. Dies ist möglicherweise darauf zurückzuführen, daß bei 105 °C im wesentlichen nur die Oberflächenfeuchte verdampft, während bei höheren Temperaturen auch die in den Poren befindliche Feuchte freigesetzt wird.

Beim thermischen Abbau des Bindemittels sollte eine Temperatur von ca. 550 °C nicht überschritten werden, da ab etwa 600 °C eine Zersetzung von Kalkstein beginnt, der vielen Baustoffen der hier interessierenden Art als Zuschlagstoff bzw. Füller beigegeben ist. Insbesondere bei Temperaturen um 900 °C treten zusätzliche Gewichtsverluste auf, die allein auf eine Zersetzung des Kalksteins in Calciumoxid und Kohlendioxid zurückgeführt werden können. Bei einer parallel zum thermischen Abbau des Bindemittels erfolgenden Zersetzung des Kalksteins oder irgendeines anderen Zuschlagstoffes würde daher sowohl die zur Bestimmung des Bindemittelgehalts vorgenommene Messung des Gewichtsverlusts der Probe als auch eine spätere Ausschlämmung zur Bestimmung des Gehalts an Zuschlagstoffen zu ungenauen Ergebnissen führen. Daher wird erfindungsgemäß vorgeschlagen, den thermischen Abbau des Bindemittels bei Temperaturen bis maximal 550 °C vorzunehmen, so daß einerseits das Bindemittel quantitativ aus der Probe entfernt und andererseits der Kalkstein unverändert in der Probe belassen werden kann.

Besonders günstige Verhältnisse ergeben sich, wenn die Vortrocknung bei einer Temperatur zwischen 105 und 300 °C und der thermische Abbau des Bindemittels bei einer Temperatur zwischen 300 und 550 °C vorgenommen wird. Dadurch wird bei der Vortrocknung sichergestellt, daß trotz einer verbesserten Trocknungswirkung der Bindemittelgehalt voll erhalten bleibt, während beim thermischen Abbau nur das Bindemittel, nicht aber auch der Kalkstein oder andere Zuschlagstoffe ganz oder teilweise aus der Probe entfernt werden. Die Dauer der Vortrocknung läßt sich auf diese Weise auf ca. drei Stunden herabsetzen, da nach dieser Zeit bereits ca. 90 Gew.% des Wasseranteils entfernt sind und daher allenfalls ein kleiner Fehler für den Bindemittelgehalt verbleibt, der aber in Kauf genommen werden kann.

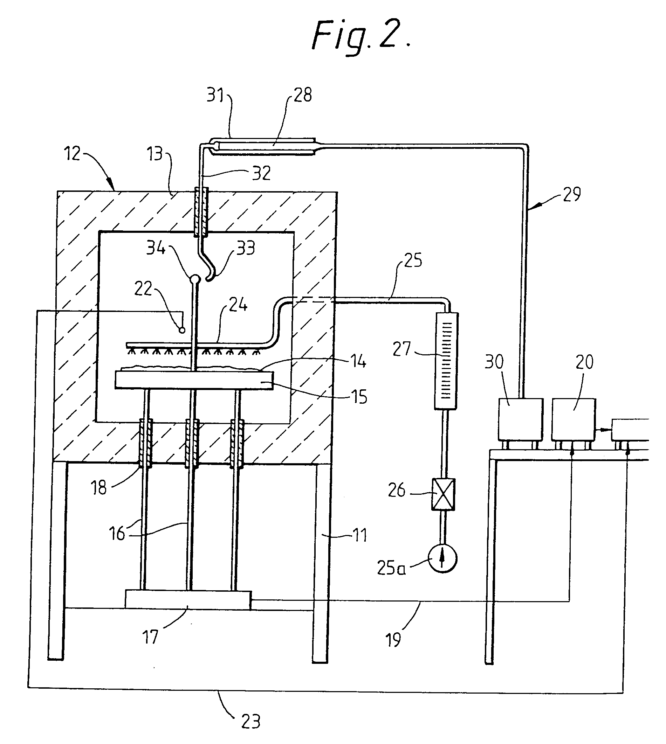

Gemäß einer bevorzugten Ausführungsform wird das erfindungsgemäße Verfahren einschließlich der Vortrockung mit einer Vorrichtung nach Fig. 2 weitgehend automatisch durchgeführt. Die Vorrichtung enthält einen auf einem Gestell 11 abgestützten Ofen 12 mit einem rundum geschlossenen Gehäuse 13, das eine nicht dargestellte Tür zum Eingeben und zur Entnahme von Proben 14 aufweist. Im Gehäuse 13 ist ein Behälter 15 angeordnet, der vorzugsweise aus einem auch bei hohen Temperaturen stabilen Metallnetz, -korb oder -gitter besteht, damit die eingegebene Probe 14 allseitig erwärmt und belüftet werden kann. Alternativ wäre möglich, einen kastenförmigen Behälter aus Edelstahl od. dgl. vorzusehen und die Probe in ein Drahtnetz od. dgl. zu verpacken. Der Behälter 15 ist auf Tragstäben 16 abgestützt, deren obere Enden in den Innenraum des Gehäuses 13 ragen, deren untere Enden auf der Lastschale oder Bodenplatte einer Waage 17 befestigt sind und deren mittlere Abschnitte in vorzugsweise aus feuerfestem und wärmedämmendem Material bestehenden Gleitbuchsen 18 verschiebbar gelagert sind. Diese Gleitbuchsen 18 sind in vertikale Bohrungen im Boden des Gehäuses 13 fest eingesetzt. Aufgrund dieser Konstruktion bilden die oberen Enden der Tragstäbe 16 eine Auflage der Waage 17 für den Behälter 15. Daher ist es möglich, das Gewicht der im Behälter 15 abgelegten Probe 14 ständig zu kontrollieren und etwaige Gewichtsverluste direkt an der Skala der Waage 17 abzulesen.

Bei der Waage 17 handelt es sich vorzugsweise um eine Waage mit elektrischer bzw. elektronischer Meßwertanzeige, bei der das mit einem Dehnungsmeßstreifen, einem Elektromagneten od. dgl. erzeugte Meßsignal in digitaler Form an einem für die Fernanzeige des Wiegeergebnisses bestimmten Ausgang abgegeben wird. Dieser Ausgang ist über eine elektrische Leitung 19 mit einem Digital/-Analog-Wandler 20 verbunden, der das digitale Meßsignal in ein analoges Signal umwandelt und an einem Ausgang abgibt, der an den entsprechenden Anschluß eines elektrisch steuerbaren x/t-Aufzeichnungsgeräts 21 angeschlossen ist, das zur automatischen analogen Aufzeichnung der von der Waage 17 abgegebenen Meßwerte in Abhängigkeit von der Zeit dient. Das Aufzeichnungsgerät 21, das ein üblicher Schreiber, ein grafikfähiger Drucker oder Plotter oder auch ein Oszillograph od. dgl. sein kann, ist vorzugsweise ein Mehrfach-Aufzeichnungsgerät mit wenigstens zwei umschaltbaren oder parallel arbeitenden Meßstellen, damit abwechselnd nacheinander oder parallel wenigstens zwei Meßgrößen aufgezeichnet werden können. Beim Ausführungsbeispiel ist ein Zweifach-Schreiber vorgesehen, dessen einer Meßstelle die vom Analog/Digital-Wandler kommenden Signale zugeführt werden.

Innerhalb des Ofens 12, dessen Temperatur geregelt weden kann, ist dicht oberhalb des Behälters 15 ein Temperaturfühler 22, z.B. ein Thermoelement, angeordnet, dessen Ausgangssignal über eine isoliert durch das Gehäuse 13 geführte elektrische Leitung 23 einer weiteren Meßstelle des Aufzeichnungsgeräts 21 zugeführt wird. Der Temperaturfühler 22 dient dazu, die in der Nachbarschaft der Probe 14 tatsächlich herrschende Temperatur laufend zu messen, während das Aufzeichnungsgerät 21 die Aufgabe übernimmt, diese Temperatur schrittweise oder kontinuierlich und analog aufzuzeichnen.

Dicht oberhalb des Behälters 15 ist ferner ein der Luftzuführung dienendes Rohr 24 mit einer Vielzahl von dem Behälter 15 zugewandten Öffnungen angeordnet, das über eine isoliert durch das Gehäuse 13 geführte Strömungsleitung 25 mit einem Druckluftanschluß 25a, einer Sauerstoffquelle od. dgl. verbunden ist. In die Strömungsleitung 25 sind außerdem zweckmäßig ein Regelventil 26 zur Einstellung der Zufuhrrate des jeweiligen Gases und ein Durchflußmesser 27 zur Ermittlung der aktuellen Durchflußrate angeordnet. Die Strömungsleitung 25 kann außerhalb des Behälters 15 schraubenlinienförmig gewunden und mit einer nicht dargestellten Heizvorrichtung ummantelt sein, um das jeweilige Gas vor seinem Eintritt in den Behälter 15 auf dessen Innentemperatur aufzuheizen. Schließlich ist oberhalb des Behälters 15 eine Exzenterwelle 28 angeordnet, die über ein schematisch angedeutetes Getriebe 29, eine Welle od. dgl. von einem Elektromotor 30 in Umdrehungen versetzt werden kann. Auf der Exzenterwelle 28 sitzt eine Hülse 31, die über ein isoliert durch das Gehäuse 13 geführtes Gestänge 32 eine Kupplung 33 trägt, die bei Bedarf mit einem Kupplungsorgan 34 des Behälters 15 gekoppelt werden kann, um diesem während der Analyse eine langsame Rüttelbewegung zu erteilen. Die Kupplung 33 besteht z.B. aus einem Haken, der in eine am Behälter 15 angebrachte Öse eingehängt und dann zusammen mit diesem angehoben werden kann.

Das erfindungsgemäße Verfahren wird bei Anwendung der Vorrichtung nach Fig. 2 auf folgende Weise ausgeführt:
Die Probe 14 wird bei einer relativ geringen Ofentemperatur von z.B. ca. 100 °C in den Ofen 12 gegeben. Die Regelvorrichtung des Ofens 12 wird auf ca. 550 °C eingestellt, so daß die Temperatur im Inneren des Behälters 15 kontinuierlich auf ca. 550 °C ansteigt und dann um diesen Wert hin- und herpendelt. Der genaue Temperaturverlauf wird dabei mittels des Temperaturfühlers 22 kontrolliert. Gleichzeitig wird der Gewichtsverlust der Probe 14 laufend mittels der Waage 17 ermittelt. Da sich dieser nach einer Verweilszeit der Probe 14 im Behälter 15 von ca. 20 - 25 min. nicht mehr ändert, kann davon ausgegangen werden, daß die thermische Zersetzung des Bindemittels nach dieser Zeitspanne abgeschlossen ist.

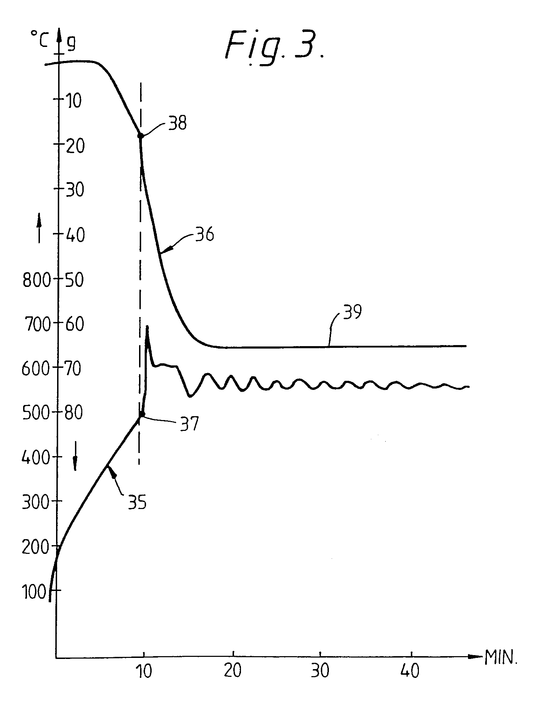

Besondere Vorteile ergeben sich dabei durch die Anwendung des Aufzeichnungsgeräts 21. In Fig. 3 ist eine mit einem üblichen Zweifachschreiber hergestellte Aufzeichnung des Analysenablaufs dargestellt. In einem kartesischen Koordinatensystem ist auf der Abszisse die Zeit in Minuten entsprechend der für den Papiervorschub od. dgl. des Aufzeichnungsgeräts 21 vorgesehenen Geschwindigkeit abgetragen, während auf der Ordinate links die Temperatur am Temperaturfühler 22 und rechts (von oben nach unten) der Gewichtsverlust der Probe 14 abgetragen ist, der auf das Anfangsgewicht der Probe 14 bei ihrer Einführung in den Behälter 15 bezogen ist.

Wie Fig. 3 zeigt, weist sowohl eine den Temperaturverlauf wiedergebende Kurve 35 als auch eine den Gewichtsverlust wiedergebende Kurve 36 jeweils einen charakteristischen Knickpunkt 37 bzw. 38 auf. Der Knickpunkt 37 wird darauf zurückgeführt, daß die Temperatur am Ort des Temperaturfühlers 22 zunächst vergleichsweise langsam ansteigt und während dieser Zeit etwa noch in der Probe 14 befindliches Wasser od. dgl. verdampft wird. Sobald jedoch das Wasser aus der Probe 14 entfernt ist (Knickpunkt 37), setzt offensichtlich schlagartig eine Verbrennung des Bindemittels ein, wodurch die Temperatur am Ort des dicht über der Probe 14 angeordneten Temperaturfühlers 22 schlagartig und kurzzeitig sogar über den zu erreichenden Wert von 550 °C ansteigt, um sich kurz danach auf den Wert von ca. 550 °C einzupendeln. Dagegen wird der Knickpunkt 38 darauf zurückgeführt, daß der bis zu seinem Erreichen auftretende Gewichtsverlust hauptsächlich auf den vergleichsweise langsamen Feuchtigkeitsverlust zurückzuführen ist, während beim Knickpunkt 38 die schlagertige thermische Zersetzung des Bindemittels einsetzt, die zu einem entsprechend schlagartig einsetzenden, vergleichsweise schnellen Gewichtsverlust der Probe 14 führt. Alle mit der Vorrichtung nach Fig. 2 vorgenommenen Messungen haben überraschend gezeigt, daß die beiden Knickpunkte 37,38 aller Proben stets zusammenfallende Abszissenwerte besitzen und daß die relativen Lagen dieser Knickpunkte im Koordinatensystem u.a. von der jeweiligen Art und Beschaffenheit der Proben 14, dem Probengewicht und den jeweiligen Versuchsbedingungen abhängen.

Was für die praktische und wirtschaftliche Anwendung des erfindungsgemäßen Verfahrens von besonderer Bedeutung ist, ist die Tatsache, daß der Knickpunkt 38 als derjenige Punkt betrachtet werden kann, bei dem die Entfernung des Wassers aus der Probe weitgehend abgeschlossen ist und die thermische Zersetzung des Bindemittels im wesentlichen beginnt. Daher können der auf die Zersetzung des Bindemittels entfallende Anteil des Gewichtsverlustes und damit auch der Bindemittelgehalt der Probe aus der Differenz zwischen dem am Knickpunkt 38 gemessenen Gewichtsverlust und demjenigen Gewichtsverlust errechnet werden, der in dem im Anschluß an die Zersetzung erreichten Bereich 39 der Kurve 36 erhalten wird und sich auch bei andauernder Aufrechterhaltung der Temperatur von ca. 550 °C praktisch nicht mehr ändert. Da der Gewichtsverlust in Fig. 3 beim Knickpunkt 38 ca. 18 g und im Bereich 39 ca. 65 g beträgt, kann daraus ein Bindemittelanteil in der Probe von ca. 47 g errechnet werden.

Eine besonders bevorzugte Variante des erfindungsgemäßen Verfahrens besteht somit darin, während der Erwärmung der Probe 14 im Ofen 12 zumindest die den Verlauf des Gewichtsverlust anzeigende Kurve 36 kontinuierlich oder schrittweise aufzuzeichnen und beim Erreichen des Bereichs 39 der Kurve 36 den auf die Zersetzung des Bindemittels entfallenden Gewichtsverlust aus der genannten Differenz zu ermitteln. Zahlreiche Versuche mit verschiedenartigen Proben, die einen bekannten Anteil an Bindemittel enthielten, haben gezeigt, daß die nach der aus Fig. 3 ersichtlichen Methode ermittelten Bindemittelgehalte mit der für praktische Zwecke erforderlichen Genauigkeit mit den bekannten Bindemittelgehalten übereinstimmen. Ein wesentlicher Vorteil besteht dabei darin, daß die Proben 14 keiner Vortrocknung unterworfen werden müssen und daß dennoch die gesuchten Bindemittelgehalte zur Verfügung stehen, sobald der Bereich 39 der Kurve 36 erreicht ist. Damit steht das Meßergebnis unabhängig von der Art und der Menge der Probe 14 jeweils sofort nach Abschluß der thermischen Zersetzung des Bindemittels, d.h. nach dem jeweils kürzest möglichen Zeitpunkt zur Verfügung.

Für eine schnelle und genaue Analyse hat es sich als zweckmäßig erwiesen, den Temperaturfühler 22 im Behälter 15 mit einem definierten Abstand von der Probe 14 anzuordnen, weil festgestellt wurde, daß sich dadurch die deutliche Ausprägung der Knickpunkte 37 und 38 günstig beeinflussen läßt. Beim dargestellten Ausführungsbeispiel hat sich ein Abstand des Temperaturfühlers 22 von der Probe 14 von ca. 15 cm als besonders zweckmäßig erwiesen.

Zur Beschleunigung der thermischen Zersetzung des Bindemittels kann während der Analyse Luft mit den angegebenen Zufuhrraten zugeführt werden, die mit dem Regelventil 26 einstellbar sind. Dabei hat sich die Anwendung eines ca. 30 cm x 30 cm großen Behälters 15 und eines über diesem angeordneten, torusförmigen Rohrs 24 als zweckmäßig erwiesen, das beispielsweise Löcher mit einem Durchmesser von 1 - 2 mm und einem Abstand von ca. 30 mm aufweist, um den Luftstrom gleichmäßig auf die gesamte Probe zu verteilen und dadurch die Zersetzung des Bindemittels wirkungsvoll zu beschleunigen. Am freien Ende ist das Rohr 24 zweckmäßig luftdicht abgeschlossen, damit die Luft nur gezielt in Richtung der Probe 14 austreten kann.

Außerdem ist es möglich, der Probe ein- oder mehrfach mechanische Energie zuzuführen, indem die Kupplungsorgane 33,34 miteinander verbunden und die Rüttelvorrichtung 28-32 eingeschaltet wird. Dabei kann sich der Rüttelvorgang in der Regel auf kurze Phasen von einigen Sekunden während der Zersetzung des Bindemittels beschränken, um dessen völlige Entfernung aus der Probe bzw. das Erreichen des Bereichs 39 der Kurve 36 zu beschleunigen. Hierfür ist es zweckmäßig, den eigentlichen Probebehälter aus einem Sieb oder Netz od. dgl. herzustellen und unter diesem eine auf der Waage 17 angeordnete Auffangschale anzuordnen, damit beim Rüttelvorgang das bereits vom Bindemittel befreite Material durch das Sieb auf die Auffangsschale fallen und dadurch dem weiteren Zersetzungsprozeß entzogen werden kann. Hierdurch wird vermieden, daß sich derartiges Material als Isolierschicht um die noch Bindemittel enthaltenden Materialteilchen legt und dadurch deren Zersetzungsvorgang behindert.

Überraschend hat sich schließlich ergeben, was für die praktische Anwendung der Erfindung von Bedeutung ist, daß die Wägung der Probe mit der Vorrichtung nach Fig. 2 ausreicht, um den Bindemittelgehalt mit der erforderlichen Genauigkeit zu bestimmen, d.h. die Probe braucht weder vor der Eingabe in den Ofen 12 noch nach der Herausnahme aus diesem und nach völliger Abkühlung zusätzlich gewogen werden. Daher kann nach Herausnahme einer Probe aus dem Ofen 12 und Abkühlung desselben auf ca. 100 bis 200 °C sofort mit der nächsten Analyse begonnen werden.

Die Erfindung ist nicht auf die beschriebenen Ausführungsbeispiele beschränkt, die sich auf vielfache Weise abwandeln lassen. Die Vortrocknung bei der Arbeitsweise nach Fig. 1 könnte beispielsweise mit Hilfe von Mikrowellen vorgenommen werden. Außerdem ist nicht erforderlich, alle Proben mit denselben Temperaturen und/oder während gleich langer Zeitintervalle zu behandeln, wenn die Arbeitsweise nach Fig. 2 und 3 gewählt wird. Ist die Probe 14 in diesem Fall bereits ausreichend vorgetrockent, entartet die Kurve 36 (Fig. 3) automatisch zu der Kurve 2 (Fig. 1). Abgesehen davon können andere Konstruktionen vorgesehen sein, die ein kontinuierliches oder schrittweises Wiegen der Probe 14 während ihrer Anordnung im Ofen 12 ermöglichen.

### Arbeitsbeispiel

Eine Probe aus frischem Mischgut (Asphaltbeton) mit einem Anfangsgewicht von 1297,3 g (einschließlich eines unbekannten Wasseranteils) wurde entsprechend Fig. 2 und 3 ohne Rüttelvorgang behandelt. Bei einem Gewichtsverlust von insgesamt 62,9 g bis zum Erreichen des Bereichs 39 lag der Knickpunkt 38 bei einem Gewichtsverlust von 15,4 g. Daraus ergibt sich, daß die Probe 15,4 g entsprechend 1,19 Gew.% Feuchtigkeit bzw. Wasser und 62,9 - 15,4 = 47,5 g entsprechend 3,66 Gew.% Bindemittel enthielt. Die Luft wurde mit einer Zuführrate von ca. 1500 l/h zugeführt. Die Analyse wurde nach zwanzig Minuten abgebrochen, da kein weiterer Gewichtsverlust mehr festgestellt werden konnte.

Das Ergebnis der Analyse stimmte mit der für praktische Anwendungszwecke ausreichenden Genauigkeit mit dem genannten Bindemittelgehalt der Probe von 3,8 Gew.% überein.

## Patentansprüche

1. Verfahren zur Ermittlung des Bindemittelgehalts von bituminösen Baustoffen, insbesondere Asphalten, bei dem das Gewicht einer Probe des Baustoffs ermittelt, das Bindemittel durch thermischen Abbau aus der Probe entfernt, das Gewicht der Probe erneut ermittelt und der Bindemittelgehalt aus dem Gewichtsverlust der Probe aufgrund der Entfernung des Bindemittels bestimmt wird, dadurch gekennzeichnet, daß die Probe beim thermischen Abbau allmählich erhitzt wird und dabei laufend eine Kurve (36) aufgezeichnet wird, die den Gewichtsverlust der Probe in Abhängigkeit von der Zeit wiedergibt, und daß nach Ablauf einer Zeitspanne, nach der die thermische Zersetzung des Bindemittels im wesentlichen abgeschlossen ist, der Bindemittelgehalt der Probe bestimmt wird aus der Differenz zwischen dem Gewichtsverlust an einem Knickpunkt (38) der Kurve (36), bei dem die Entfernung der Feuchtigkeit aus der Probe abgeschlossen ist und die thermische Zersetzung des Bindemittels beginnt, und demjenigen Gewichtsverlust, der in dem im Anschluß an die Zersetzung erreichten Bereich (39) der Kurve (36) erhalten wird.

2. Verfahren nach Anspruch 1, dadurch gekennzeichnet, daß die Probe zum thermischen Abbau des Bindemittels auf 500° bis 550°C erhitzt wird.

3. Verfahren nach Anspruch 1 oder 2, dadurch gekennzeichnet, daß der Probe während des thermischen Abbaus des Bindemittels mechanische Energie zugeführt wird.

4. Verfahren nach einem der Ansprüche 1 bis 3, dadurch gekennzeichnet, daß der Probe während des thermischen Abbaus des Bindemittels zusätzlicher Sauerstoff zugeführt wird.

## Claims

1. Method for determining the binding materials content of bituminous road-making materials, in particular asphalts, wherein the weight of a sample of the road-making material is determined, the binding material is removed from said sample by thermal separation, the weight of said sample is determined once again and the content of said binding material is calculated from the weight loss of said sample after removal of said binding material, characterised in that in the thermal separation process, said sample is heated gradually, whereby a curve (36) is continuously plotted to indicate the weight loss of said sample as a function of time; and that, after a certain time interval, after which the thermal decomposition of the binding material has essentially been concluded, the content of the binding material in said sample is determined from the difference between the weight loss at a salient point (38) in the curve (36), at which removal of moisture from said sample has concluded and the thermal decomposition of the binding material begins, and the weight loss obtained in the area (39) of the curve (36) reached subsequent to decomposition.

2. Method according to Claim 1, characterised in that for the thermal separation process, the sample is heated to 500° - 550°C.

3. Method according to Claim 1 or 2, characterised in that mechanical energy is supplied to the sample during thermal separation of the binding material.

4. Method according to one of Claims 1 to 3, characterised in that additional oxygen is supplied to the sample during thermal separation of the binding material.

## Revendications

1. Procédé de détermination de la teneur en liants de matériaux de construction bitumineux, d'asphaltes notamment, dans lequel le poids d'un échantillon du matériau est déterminé, le liant est éliminé de l'échantillon par dégradation thermique, le poids de l'échantillon est redéterminé, et la teneur en liant est établie à partir de la perte de poids de l'échantillon due à l'élimination du liant, caractérisé en ce que l'échantillon est chauffé progressivement lors de la dégradation thermique et une courbe (36), qui reproduit la perte de poids de l'échantillon en fonction du temps, est alors enregistrée en continu, et en ce que, après l'écoulement d'un intervalle de temps, au bout duquel la décomposition thermique du liant est essentiellement terminée, la teneur en liant de l'échantillon est déterminée à partir de la différence entre la perte de poids sur un point d'inflexion (38) de la courbe (36), pour lequel l'élimination de l'humidité de l'échantillon est terminée et la décomposition thermique du liant commence, d'une part, la perte de poids, établie dans la zone (39) de la courbe (36), obtenue à la suite de la décomposition, d'autre part.

2. Procédé suivant la revendication 1, caractérisé en ce que l'échantillon est chauffé à 500 - 550°C pour la dégradation thermique du liant.

3. Procédé suivant l'une des revendications 1 et 2, caractérisé en ce que l'échantillon est alimenté en énergie mécanique pendant la dégradation thermique du liant.

4. Procédé suivant l'une quelconque des revendications 1 à 3, caractérisé en ce que l'échantillon est alimenté en oxygène supplémentaire pendant la dégradation thermique du liant.
